Europäisches Patentamt

European Patent Office

Office européen des brevets

(19)

(11) Veröffentlichungsnummer: **0 041 654**
**B1**

(12) **EUROPÄISCHE PATENTSCHRIFT**

(45) Veröffentlichungstag der Patentschrift:
**21.09.83**

(51) Int. Cl.³: **A 01 N 25/24,** A 61 K 7/40

(21) Anmeldenummer: **81104086.4**

(22) Anmeldetag: **27.05.81**

(54) Mittel zur Bekämpfung tierischer Ektoparasiten mit stark ausgeprägter Residualwirkung.

(30) Priorität: **10.06.80 DE 3021725**

(43) Veröffentlichungstag der Anmeldung:
**16.12.81 Patentblatt 81/50**

(45) Bekanntmachung des Hinweises auf die Patenterteilung:
**21.09.83 Patentblatt 83/38**

(84) Benannte Vertragsstaaten:
**BE DE FR GB IT NL SE**

(56) Entgegenhaltungen:
**DE-A-2 658 725**
**FR-A-2 229 351**
**GB-A-1 108 837**
**GB-A-1 427 451**

(73) Patentinhaber: **BAYER AG, Zentralbereich Patente, Marken und Lizenzen, D-5090 Leverkusen 1, Bayerwerk (DE)**

(72) Erfinder: **Dorn, Hubert, Dr., Pahlkestrasse 71, D-5600 Wuppertal 1 (DE)**
Erfinder: **Stendel, Wilhelm, Dr., In den Birken 55, D-5600 Wuppertal 1 (DE)**
Erfinder: **Voege, Herbert, Dr., Martin-Buber-Strasse 41, D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Mittel zur Bekämpfung tierischer Ektoparasiten mit stark ausgeprägter Residualwirkung

Die Erfindung betrifft neue Mittel mit stark ausgeprägter Residualwirkung über einen längeren Zeitraum zur Bekämpfung tierischer Ektoparasiten, vorzugsweise zur Bekämpfung von Insekten.

Es ist in der Vergangenheit bereits wiederholt versucht worden, bei insektiziden Substanzen z.B. Phosphorsäureesterverbindungen durch spezielle Formulierungen den Zeitraum der Wirksamkeit zu verlängern, d.h. eine gute Residualwirkung zu erzielen. Dies geschah und geschieht z.B. dadurch, dass flüchtige insektizide Verbindungen mikroverkapselt werden. Auch das Einarbeiten von Insektiziden in Polymere, aus denen sie über einen längeren Zeitraum verdampfen, wird in der Praxis durchgeführt, z.B. bei Hundehalsbändern oder bei Fliegen-Strips mit beispielsweise O,O-Dimethyl-O-(2,2-dochlorvinyl)-phosphorsäureester als Wirkstoff. Zur Bekämpfung von tierischen Ektoparasiten und speziell von Insekten am Grosstier sind solche Verfahren bisher nur selten zum Einsatz gekommen.

So wurden zum Beispiel insektizide Ohrmarken gegen Zecken, die das Ohr bevorzugen, getestet. (E.H. Ahrens et al., J. of Econom. Entomology, 70, 581–585).

Gegen Hydrotea irritans wurden bei Schafen insektizide Teerformulierungen geprüft, die gegenüber der Reinsubstanz eine verlängerte Wirksamkeit aufwiesen (R.N. Titchener, A.D. Herlyn and I.W. Newbold, Proceedings of the 8th Insecticide Conference 1975, 533–538).

Des weiteren sind Repellent-Formulierungen mit thermoplastischen Harzen beschrieben worden, die, auf die Haut aufgebracht, die Repellent-Wirkung verlängern (GB-PS 1 322 805).

Insektizide Formulierungen mit N-Vinyl-Lactam-Polymeren oder Vinylacetat-Crotonsäure-Copolymeren zur Anwendung bei Mensch und Tier werden ebenfall in der Literatur beschrieben (z.B. US-PS 3 301 808, GB-PS 1 427 451).

Zur Anwendung am Tier wurde auch eine insektizide Formulierung mit Haftkleberzusatz angemeldet (DE-OS 26 58 725).

Das in der genannten DE-OS beschriebene Verfahren ist auf die Bekämpfung symboviner Fliegen gerichtet, weil speziell beim Rind dieses Indikationsgebiet in letzter Zeit grosse Bedeutung erlangt hat. Hierfür sind vor allem zwei Gründe verantwortlich:

a. Neue Methoden der Massenhaltung von Rindern (feed lots usw)
b. Verbot des Einsatzes des gegen Fliegen stark wirksamen Stoffes DDT bei schlachtbaren Tieren.

Obwohl eine Reihe von Fliegenmitteln für Grosstiere zur Zeit erhältlich ist, war die wirksame und vor allem dauerhafte Bekämpfung der symbovinen Fliegen beim Rind bis jetzt immer noch ein ungelöstes Problem.

Die Ursache ist in dem ungenügenden Residualeffekt der bisher gegen symbovine Fliegen eingesetzten Präparate zu suchen. Somit ist das Problem der Fliegen-Bekämpfung beim Rind weniger ein Problem des Wirkstoffeinsatzes als vielmehr einer geeigneten langwirksamen Formulierung. Ähnliche Probleme treten bei anderen Grosstieren auf.

Eine langwirkende Formulierung sollte sowohl auf nassen wie auf trockenen Tieren angewendet werden können. Dies ist insofern wichtig, da in tropischen Ländern die Rinder in der Regel zur Zeckenbekämpfung gebadet werden. Im Anschluss an dieses Bad wäre es vorteilhaft, die Tiere nun auch noch mit einem Schutz gegen symbovine Fliegen zu versehen. Zum anderen sollte eine solche Formulierung auch auf trockenen Tieren anzuwenden sein.

Bei den zuvor zitierten insektiziden Formulierungen zum Auftragen auf das Haarkleid ist eine Anwendung aber nur bei trockenen Tieren möglich. Die Langzeitwirkung ist dabei nicht ausreichend.

Es wurden Formulierungen mit insektizider und/oder Repellent-Wirkung gefunden, die eine ausgezeichnete und auch für die Bekämpfung symboviner Fliegen ausreichende Residualwirkung aufweisen und auf nassem wie trockenem Haarkleid haften.

Die Erfindung betrifft daher insektizide Formulierungen mit ausgeprägter Residual-Wirkung, die sowohl auf nasse wie trockene Tiere aufgetragen werden können, dadurch gekennzeichnet, dass sie:

a. 0,1–20%, vorzugsweise 0,1–5% eines insektiziden Wirkstoffes

b. 1–40%, vorzugsweise 1–20% eines wasserlöslichen Gel- oder Lackbildenden Polymers

c. 40–98%, vorzugsweise 60–90% eines organischen, wassermischbaren Lösungsmittels, das schneller verdunstet als Wasser und in dem sich das Polymer nicht löst

d. 0,1–10%, verschiedener Additiva, z.B. Weichmacher, Suspendierhilfsmittel, Antioxydantien, Farbstoffe etc.

enthalten.

Zur Herstellung der Formulierung werden an sich bekannte Polymere oder deren Salze in einem Lösungsmittel suspendiert, in dem sie nicht löslich sind. Die Polymeren verquellen dagegen in Wasser zu einem Gel. Der Wirkstoff wird in dem Lösungsmittel entweder suspendiert oder gelöst. Das Lösungsmittel muss mit Wasser mischbar sein und schneller als Wasser verdunsten können. Der Suspension können die üblichen Formulierhilfsmittel zugesetzt werden, um eine leicht aufschüttelbare oder homoge Suspension sicherzustellen. Ein Weichmacherzusatz kann ebenfalls erwünscht sein, um den sich bildenden Film später elastisch zu halten.

Wird eine solche Suspension auf ein nasses Tier gegossen oder gesprüht, so verquillt das Polymer mit dem Verdunsten des Lösungsmittels zu einem Gel, das zu einer Lack- oder Filmschicht austrocknet und dabei den Wirkstoff inkorporiert. Diese Schicht bleibt lange Zeit auf dem Haarkleid haften und wird durch Regengüsse oder ein Dipbad nur langsam – nach und nach – abgewaschen.

Ist das Tier trocken oder soll mit der Suspension z.B. eine Stallwand besprüht werden, empfiehlt sich folgende Arbeitsweise:

Die Suspension wird im etwa gleichen Verhältnis mit Wasser verdünnt. Sie ist dann noch niedrigviskos, das Polymer noch nicht verquollen, so dass sie sich mit den üblichen Geräten mühelos applizieren lässt. Auch hier bildet sich nach dem Verdunsten des Lösungsmittels ein Gel und später ein Film aus, wie bereits oben beschrieben.

Als Wirkstoffe kommen folgende Insektizide zur Anwendung: Aus der Gruppe der Phosphorsäureester z.B. Coumaphos, Fenthion, Trichlorfon, Naled, Bromophos, Famphur, Stirofos, Diazinon. Aus der Gruppe der Carbamate z.B. Propoxur, Croneton, Carbaryl. Weiter chlorierte Insektizide Verbindungen wie Toxaphen, Formamidin und dessen Derivate wie z.B. 2-methyl-1,3-bis[2,4-dimethylphenylimino]-2-aza-propan. Insbesondere eignen sich auch die Gruppen der synthetischen Pyrethroide und insektizide Thioharnstoffe oder Harnstoffe.

Die insektiziden Wirkstoffe können in ihrer Wirksamkeit durch Synergisten verstärkt werden, wie z.B. Piperonylbutoxid oder Sesamex.

Auch Repellents können in diese Formulierungen eingearbeitet werden, z.B. Diethyltoluamid, Dibutylphthalat, 2-(Oktylthio)-ethanol.

Auch «Lockstoffe» können der Formulierung zusammen mit Insektiziden beigefügt werden. Die symbovinen Fliegen werden vermehrt zu einem behandelten Tier gelockt und werden dort getötet, so dass nur ein Teil der Herde behandelt werden muss. Solche Lockstoffe sind z.B.: Vanillin, (Z)-9-tricosene, (Z)-8-Dodecenylacetat, Oktylbutyrat, Eugenol.

Als Gel- und Filmbildner kommen – wie gesagt – alle makromolekularen Verbindungen in Frage, die sich in dem wassermischbaren, organischen Lösungsmittel nicht lösen und nach Mischen mit Wasser zu einem Gel verquellen, das nach dem Trocknen eine Art Film gibt.

Folgt man einer Einteilung der makromolekularen Hilfsstoffe, wie z.B. von Keipert et al. in Die Pharmazie 28, 145–183 (1973) beschrieben, so kommen vor allem ionische Makromoleküle in deren Salzform zur Anwendung. Dies sind unter anderem Natriumcarboxymethylcellulose, Polyacrylsäure, Polymethacrylsäure und deren Salze, Natriumamylopektinsemiglykolat, Alginsäure und Propylenglykol-Alginat als Natriumsalz, arabisches Gummi, Xanthan-Gummi, Guar-Gummi.

Amphotere Makromoleküle wie Protein-Derivate, z.B. Gelatine sind ebenso geeignet wie nichtionische Polymere z.B. Methylcellulose, andere Cellulose-Derivate und lösliche Stärken, die obige Anforderungen erfüllen.

Als Lösungsmittel sind alle mit Wasser mischbaren Flüssigkeiten geeignet, die das Makromolekül nicht lösen und schneller verdunsten als Wasser.

In Betracht kommen z.B. Alkanole wie Ethanol und Isopropylalkohol; Ketone wie Aceton, Methylethylketon, Glykolether wie Ethylenglykolmonomethylether oder -ethylether.

Es können bei der Herstellung der erfindungsgemässen pour-on-Formulierungen ein oder mehrere Lösungsmittel eingesetzt werden.

Als weitere Hilfsmittel sind geeignet:

a. Substanzen, die die Suspension stabilisieren können, z.B. kolloidale Kieselsäure, Montmorillonite u.a.

b. Tenside (beinhaltet Emulgatoren und Netzmittel), z.B.
1. anionaktive, wie Na-Laurylsulfat, Fettalkoholethersulfate, Mono/Dialkylpolyglykolether-orthophosphorsäureester-Monoethanolaminsalz;
2. kationaktive, wie Cetyltrimethylammoniumchlorid;
3. ampholytische, wie Di-Na-N-lauryl-$\beta$-iminodipropionat oder Lecethin;
4. nicht ionogene, z.B. polyoxethyliertes Rizinusöl, polyoxyethyliertes Sorbitan-Monooleat, Sorbitan-Monostearat, Cetylalkohol, Glycerinmonostearat, Polyocyethylenstearat, Alkylphenolpolyglykolether.

c. Stabilisatoren zur Verhinderung des bei einigen Wirkstoffen eintretenden chemischen Abbaues wie Antioxydantien, z.B. Tocopherole, Butylhydroxyanisol.

d. Weichmacher für die Elastizität der Filmbildner, z.B. Glycerin, Propylenglykol.

Beispiel 1
(Demonstration der Haftwirkung am Tier)
Eine Formulierung wurde mit gelbem Eisenoxid zur Farb-Markierung nach folgender Zusammensetzung hergestellt:

| | |
|---|---|
| Na-alginat | 10,0 g |
| kolloidale Kieselsäure | 5,0 g |
| Eisenoxid Pigmentgelb | 2,0 g |
| Isopropanol ad | 100 ml |

Es wurden zwei Rinder völlig durchnässt. Obige Suspension wurde einmal aufgegossen und einmal mit einer Gartenspritze aufgesprüht. Nach dem Trocknen der Tiere bildet sich eine fest haftende Masse. Die Tiere wurden nun 1 mal täglich längere Zeit mit dem Schlauch erneut «beregnet». Nach 8 Tagen war immer noch deutlich die Haftmasse zu sehen.

Beispiel 2
In einem biologischen Modellversuch wurde die Haftwirkung einer erfindungsgemässen Formulierung im Vergleich zur Haftwirkung einer herkömmlichen Emulsionskonzentrat-Formulierung geprüft.

Dabei wurde wie folgt vorgegangen: die Konzentrate werden durch Zugabe von Wasser auf die jeweils gewünschte Anwendungskonzentration verdünnt.

Je 1 ml der zu testenden Anwendungskonzentration wird in eine Petrischale (∅ 10 cm) pipettiert und so verteilt, dass sich ein dünner Flüssigkeitsfilm bildet. Danach wird dieser Film 24 h an der Luft getrocknet.

Nach Ablauf dieser Zeit werden je 2 der Petrischalen pro Konzentration mit angetrocknetem Anwendungskonzentrationsfilm bis zum Testbeginn in diesem Zustand belassen. Je 2 weitere Petrischalen je Konzentration, die ebenfalls über einen Zeitraum von 24 h getrocknet waren, werden nun 15' bzw. 60' lang unter schwach fliessendem Wasser abgespült und danach erneut 24 h an der Luft getrocknet.

Nun werden auf die so vorbereiteten Petrischalen mit den Anwendungsverdünnungen adulte Fliegen der Art Stomoxys calcitrans gebracht. Es wird festgestellt, welche Konzentration innerhalb von 5 Minuten die aufgesetzten Fliegen sicher abtötet. Falls sich die zur Abtötung notwendige Konzentration durch das Abspülen des Wirkstoffes verändert, wird angenommen, dass der Wirkstoff aus dem Film herausgewaschen wurde, während ein Gleichbleiben der zur Wirkung notwendigen Konzentration ein Verbleiben des Wirkstoffes im Wirkstoffilm beweist.

Die erhaltenen Befunde des Vergleichsversuches gehen aus folgender Tabelle hervor:

Die Zahlen in der Tabelle geben die Wirkstoffkonzentration in ppm an, die zur Abtötung der Fliegen (Stomoxys calcitrans) innerhalb von 5 Minuten notwendig ist.

| Petrischale mit Wirkstoffilm | herkömmliche Formulierung | erfindungsgemässe Formulierung |
|---|---|---|
| trocken | 1 | 30 |
| Abspülzeit 15' | 10 | 30 |
| Abspülzeit 60' | 1000 | 30 |

## Patentansprüche

1. Insektizide Formulierungen mit ausgeprägter Residual-Wirkung, die sowohl auf nasse wie trockene Tiere aufgetragen werden können, dadurch gekennzeichnet, dass sie:

0,1–20% eines insektiziden Wirkstoffes,
1–40% eines wasserlöslichen Gel- oder Lackbildenden Polymers,
40–98% eines organischen, wassermischbaren Lösungsmittels, das schneller verdunstet als Wasser und in dem sich das Polymer nicht löst, und
0,1–10% verschiedener Additiva, wie Weichmacher und/oder Suspendierhilfsmittel und/oder Antioxydantien und/oder Farbstoffe enthalten.

2. Insektizide Formulierungen nach Anspruch 1, dadurch gekennzeichnet, dass sie 0,1–5% eines insektiziden Wirkstoffes, 1–20% eines wasserlöslichen Gel- oder Lackbildenden Polymers, 60–90% eines organischen, wassermischbaren Lösungsmittels, das schneller verdunstet als Wasser und in dem sich das Polymer nicht löst, enthalten.

3. Insektizide Formulierungen nach Anspruch 1, dadurch gekennzeichnet, dass sie als wasserlösliches Gel- oder Lackbildendes Polymer Natriumcarboxymethylcellulose, Polyacrylsäure, Polymethacrylsäure, Natriumamylopektinsemiglykolat, Alginsäure, Propylenglykol-Alginat, arabisches Gummi, Xanthan-Gummi oder Guar-Gummi enthalten, wobei die ionischen Makromoleküle in deren Salzform zur Anwendung gelangen.

4. Insektizide Formulierungen nach Anspruch 1, dadurch gekennzeichnet, dass sie als wasserlösliches Gel- oder Lackbildendes Polymer amphotere Makromoleküle, Cellulose-Derivate oder lösliche Stärken enthalten.

5. Insektizide Formulierungen nach Anspruch 1, dadurch gekennzeichnet, dass sie als Wirkstoff einen Phosphorsäureester, ein synthetisches Pyrethroid oder einen insektiziden Thioharnstoff oder Harnstoff enthalten.

6. Insektizide Formulierungen nach Anspruch 5, dadurch gekennzeichnet, dass sie ein Repellent enthalten.

7. Insektizide Formulierungen nach Anspruch 5, dadurch gekennzeichnet, dass sie einen Insekten-Lockstoff enthalten.

8. Verwendung von insektiziden Formulierungen gemäss Anspruch 1 zur Bekämpfung von Insekten, dadurch gekennzeichnet, dass man die Formulierung auf trockene oder nasse Tiere durch Aufgiessen oder Besprühen aufbringt.

## Claims

1. Insecticidal formulations which have a pronounced residual action and can be applied both to wet animals and to dry animals, characterised in that they contain:

0.1–20% of an insecticidal active compound,
1–40% of a water-soluble gel-forming or lacquer-forming polymer,
40–98% of an organic, water-miscible solvent which evaporates more rapidly than water and in which the polymer does not dissolve, and
0.1–10% of various additives, such as plasticisers and/or suspending auxiliaries and/or antioxidants and/or dyestuffs.

2. Insecticidal formulations according to Claim 1, characterised in that they contain 0.1–5% of an insecticidal active compound,

1–20% of a water-soluble gel-forming or lacquer-forming polymer,

60–90% of an organic, water-miscible solvent which evaporates more rapidly than water and in which the polymer does not dissolve.

3. Insecticidal formulations according to Claim 1, characterised in that they contain sodium carboxymethylcellulose, polyacrylic acid, polymethacrylic acid, sodium amylopectin semiglycolate, alginic acid, propylene glycol alginate, gum arabic, xanthan gum or guar gum as the water-soluble gel-forming or lacquer-forming polymer, the ionic macromolecules being employed in salt form.

4. Insecticidal formulations according to Claim 1, characterised in that they contain amphoteric macromolecules, cellulose derivatives or soluble starches as the water-soluble gel-forming or lacquer-forming polymer.

5. Insecticidal formulations according to Claim 1, characterised in that they contain a phosphoric acid ester, a synthetic pyrethroid or an insecticidal thiourea or urea as the active compound.

6. Insecticidal formulations according to Claim 5, characterised in that they contain a repellent.

7. Insecticidal formulations according to Claim 5, characterised in that they contain an insect bait.

8. Use of insecticidal formulations according to Claim 1 for combating insects, characterised in that the formulation is applied by pouring or spraying it on to dry or wet animals.

**Revendications**

1. Formulations insecticides à action rémanente prononcée, qui peuvent être appliquées aussi bien sur des animaux mouillés que sur des animaux secs, caractérisées en ce qu'elles contiennent:

0,1–20% d'une substance active insecticide,
1–40% d'un polymère hydrosoluble formant un gel ou une laque,
40–98% d'un solvant organique miscible à l'eau qui s'évapore plus rapidement que l'eau et dans lequel le polymère n'est pas soluble, et

0,1–10% de différents additifs tels que des plastifiants et/ou des adjuvants de mise en suspension et/ou des antioxydants et/ou des colorants.

2. Formulations insecticides suivant la revendication 1, caractérisées en ce qu'elles contiennent

0,1–5% d'une substance active insecticide,
1–20% d'un polymère hydrosoluble formant un gel ou une laque,
60–90% d'un solvant organique miscible à l'eau qui s'évapore plus rapidement que l'eau et dans lequel le polymère n'est pas soluble.

3. Formulations insecticides suivant la revendication 1, caractérisées en ce qu'on utilise comme polymère hydrosoluble formant un gel ou une laque, le sel de sodium de la carboxyméthylcellulose, l'acide polyacrylique, l'acide polyméthacrylique, l'amylopectine-semiglycolate de sodium, l'acide alginique, l'alginate de propylèneglycol, la gomme arabique, la gomme xanthane ou la gomme guar, les macromolécules ioniques étant utilisées sous leur forme de sel.

4. Formulations insecticides suivant la revendication 1, caractérisées en ce qu'elles contiennent comme polymère hydrosoluble formant un gel ou une laque des macromolécules amphotères, des dérivés cellulosiques ou des amidons solides.

5. Formulations insecticides suivant la revendication 1, caractérisées en ce qu'elles contiennent comme substance active un ester d'acide phosphorique, une pyréthrine synthétique ou une thiourée ou urée insecticide.

6. Formulations insecticides suivant la revendication 5, caractérisées en ce qu'elles contiennent un répulsif.

7. Formulations insecticides suivant la revendication 5, caractérisées en ce qu'elles contiennent un attractif sexuel pour les insectes.

8. Utilisation de formulations insecticides suivant la revendication 1 dans la lutte contre des insectes, caractérisée en ce qu'on applique la formulation en la versant ou par aspersion sur des animaux secs ou mouillés.